(19) Europäisches Patentamt
European Patent Office
Office européen des brevets

(11) EP 3 725 154 B1

(12) **FASCICULE DE BREVET EUROPEEN**

(45) Date de publication et mention de la délivrance du brevet:
**12.08.2026 Bulletin 2026/33**

(21) Numéro de dépôt: **20166050.3**

(22) Date de dépôt: **09.04.2015**

(51) Classification Internationale des Brevets (IPC):
***A01N 37/04*** *(2006.01)* ***A01N 37/36*** *(2006.01)*
***A01N 37/42*** *(2006.01)* ***A01P 1/00*** *(2006.01)*
***A01P 3/00*** *(2006.01)* ***A61K 8/365*** *(2006.01)*
***A61Q 19/10*** *(2006.01)* ***A61K 8/36*** *(2006.01)*
***A61K 8/60*** *(2006.01)* ***A61Q 17/00*** *(2006.01)*

(52) Classification Coopérative des Brevets (CPC):
(C-Sets disponibles)
**A01N 37/36; A01N 37/04; A01N 37/42;
A61K 8/361; A61K 8/365; A61K 8/602;
A61Q 17/005; A61Q 19/10;** A61K 2800/596
(Cont.)

(54) **NOUVEAUX PRODUITS BIOCIDES**
NEUARTIGE BIOZIDE PRODUKTE
NOVEL BIOCIDAL PRODUCTS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **10.04.2014 FR 1453179**

(43) Date de publication de la demande:
**21.10.2020 Bulletin 2020/43**

(62) Numéro(s) de document de la (des) demande(s) initiale(s) en application de l'article 76 CBE:
**15720212.8 / 3 128 839**

(73) Titulaire: **Salveco**
**88100 Saint Die Des Vosges (FR)**

(72) Inventeur: **AUBERGER, Stephan**
**88100 Neuvillers sur Fave (FR)**

(74) Mandataire: **Cabinet Bleger-Rhein-Poupon**
**4a rue de l'Industrie**
**67450 Mundolsheim (FR)**

(56) Documents cités:
**WO-A1-2012/114039**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 725 154 B1

Processed by Luminess, 75001 PARIS (FR)

**(Cont. page suivante)**

(52) Classification Coopérative des Brevets (CPC):
(Cont.)

C-Sets
**A01N 37/04, A01N 25/02, A01N 25/04, A01N 25/30, A01N 25/34, A01N 2300/00;**
**A01N 37/36, A01N 25/02, A01N 25/04, A01N 25/30, A01N 25/34, A01N 2300/00;**
**A01N 37/42, A01N 25/02, A01N 25/04, A01N 25/30, A01N 25/34, A01N 2300/00**

## Description

La présente invention porte sur une nouvelle génération de produits biocides qui ne présentent pas de risques chroniques et très peu d'impacts aigus pour la santé et l'environnement.

En effet, les substances actives et les adjuvants qui composent les formulations biocides selon l'invention sont d'origine végétale, de préférence agricole, et renouvelable, et sont par ailleurs totalement, naturellement et rapidement biodégradables.

Les différentes applications de ces nouveaux produits biocides sont également revendiquées.

Les produits biocides sont communément utilisés dans une large variété de produits, incluant des désinfectants ménagers et/ou industriels, des insecticides, des produits de traitement du bois, etc.

Ces produits peuvent être utilisés pour nettoyer et désinfecter tous types de surfaces, y compris la peau.

Les produits biocides sont destinés à détruire, repousser ou rendre inefficaces les organismes indésirables, jugés nuisibles ou non, comme notamment les bactéries, les virus, les champignons, à en prévenir l'action ou à les combattre de toute autre manière par une action chimique ou biologique.

Ces catégories de produits sont des produits actifs sur le plan chimique ou biologique, et, de ce fait, sont susceptibles d'avoir des effets nuisibles sur l'homme, l'animal et/ou l'environnement.

Plus particulièrement, un biocide se caractérise en général par son champ d'application : bactéricide, fongicide, antiviral et/ou insecticide.

Les bactéricides ont une action contre certaines bactéries Gram+ et/ou Gram-. Les fongicides ont une action contre certains champignons et certaines levures. En ce qui concerne les antiviraux, ceux-ci ont une action contre certains virus.

Les actifs biocides peuvent donc présenter un risque majeur pour la santé humaine, animale mais également pour l'environnement ; d'ailleurs, d'un point de vue étymologique « bios » signifie « la vie » et « -cide » signifie « qui tue ». En effet, les actifs biocides peuvent agir longtemps, se diffuser, être partiellement dégradés, ou ne pas être dégradés du tout suivant leur nature, et ils sont alors considérés comme (bio)accumulables et toxiques.

Les substances actives qui composent les biocides sont en général des principes actifs d'origine pétrochimique.

Quelques rares substances d'origine végétales peuvent être répertoriées, comme de l'éthanol ou des huiles essentielles ayant des propriétés bactériostatiques et limitées. De plus, ces substances sont hors marchés professionnels.

Enfin, les biocides peuvent également comporter un mélange des deux dernières catégories de composants, c'est-à-dire des principes actifs d'origine pétrochimique et des substances d'origine végétale, dans le but d'atteindre l'efficacité souhaitée.

On connait ainsi, dans l'état de la technique, les dérivés d'ammonium quaternaires qui représentent une classe majeure des substances biocides d'origine chimique qui sont utilisées dans les désinfectants de surface et pour l'hygiène des mains.

D'autres substances sont également utilisées comme l'hypochlorite de sodium (Eau de Javel), les di- ou tri-amines ou le 5-chloro-2-(2,4 dichlorphenoxy)phenol (Triclosan®).

Leur classification réglementaire au regard de leurs effets préoccupants, corrosifs, sensibilisants et très toxiques pour les organismes aquatiques, de même que la suspicion d'effet CMR (Cancérigène, Mutagène, Reprotoxique) et de perturbateur endocrinien de certains représentants de cette classe, impose la plus grande prudence à l'utilisation de ces produits.

Une prise de conscience internationale à ce sujet a abouti à l'adoption de Règlements européens (REACH : 1907/2006 et Biocides : 528/2012) ayant pour objectif de limiter la commercialisation de substances chimiques dangereuses et d'en réguler strictement l'utilisation.

En effet, le marché des produits biocides pour l'Europe représente un volume de 300 000 à 750 000 tonnes par an (Étude de l'impact de la directive Biocides 98/8/EC - 2007) ce qui correspond à un marché mondial annuel de 7 milliards d'euro.

Il y a donc un intérêt considérable développer de nouveaux produits biocides pour lutter contre des micro-organismes indésirables. Ces biocides devront avoir la capacité d'être intégralement dégradés de façon naturelle après leur utilisation, de sorte à diminuer les risques pour l'homme, l'animal et l'environnement, tout en limitant les résistances induites.

En particulier, pour l'homme et l'animal, ces risques peuvent être « directs », liés à la toxicité dite aigüe, et surtout « indirects », liés à la toxicité chronique.

En outre, les produits biocides doivent être efficaces à faible concentration de façon à proposer des solutions industrielles et économiques conformes aux positionnements économiques des marchés sanitaires.

Afin de proposer des produits biocides qui ne soient pas toxiques et/ou préjudiciables pour l'homme et l'environnement, et qui soient également biodégradables et biocompatibles pour l'homme, des biocides d'origine naturelle ont alors été développés.

En effet, les produits biocides d'origine végétale semblent les plus appropriés pour répondre à ces objectifs combinés.

La majorité des solutions biocides d'origine végétale contiennent une base lavante combinée à une base désinfectante. Cependant, ces produits ne présentent pas la même efficacité biocide ni la même facilité d'utilisation (rapport dose / temps d'action / spectre d'action) que les solutions biocides contenant des actifs et/ou des adjuvants chimico-synthétiques.

Il y a donc une nécessité de développer une alternative durable et sécuritaire à l'utilisation de produits chimico-synthétiques, et qui permette d'obtenir une efficacité biocide et une facilité d'usage de produit à diluer.

On connait déjà, par le document de brevet WO 2012/114039 une composition biocide comprenant :

- entre 0,01% et 20% d'agent chélatant ;
- entre 0,03% et 25% de tensioactifs non-ioniques de type polyols et de préférence entre 0,03% et 25% de tensioactifs non-ioniques de type glycosides, esters de polyglycérols ou esters de sorbitan ;
- entre 0,03% et 25% de tensioactifs anioniques, de préférence de type carboxylates ou polycarboxylates ;
- entre 0,1% et 75% d'un ou plusieurs acides organiques ;
- entre 0,001% et 8% de parfum naturel.

Toutefois, cette composition, bien que présentant une origine végétale et ayant une activité biocide sur certains pathogènes, peut encore être améliorée.

En effet, la composition décrite dans ce document ne présente pas une efficacité optimale à l'encontre d'organismes indésirables et susceptibles d'être pathogènes, par exemple certaines souches de champignons ou encore certains virus indésirables ou pathogènes.

La présente invention porte ainsi sur une nouvelle génération de formulations biocides pour le nettoyage et la désinfection de tous types de surfaces.

Leur impact sur l'environnement est très limité, voir nul, et dans tous les cas il n'est pas considéré comme préoccupant. Cela résulte des données toxicologiques des composants et du mélange, qui sont significativement inférieures aux seuils critiques de classification imposées.

En effet, ces formulations biocides sont issues de ressources végétales renouvelables, de préférence des ressources agricoles, et elles sont intégralement biodégradables sur l'ensemble de la formulation.

La substance active des formulations biocides selon l'invention consiste en au moins un acide organique d'origine végétale. Cet acide organique peut être issu directement d'une ressource végétale, de préférence agricole, ou d'une ressource agricole transformée.

La présente invention décrit une composition liquide aqueuse à effet virucide à l'encontre de souches de virus appartenant à la famille de adénovirus ou à effet virucide à l'encontre de rotavirus ou à effet fongicide à l'encontre de champignons appartenant à l'espèce *Aspergillus niger,* ladite composition comprenant une formulation biocide concentrée biodégradable et étant conforme à l'objet de la revendication indépendante 1.

De manière avantageuse, la proportion en acide organique dans la formulation biocide est conforme à l'objet de la revendication dépendante 4. Pour ce qui est de la proportion en tensioactif anionique, celle-ci est conforme à l'objet de la revendication dépendante 4, de préférence conforme à l'objet de la revendication dépendante 5.

La formulation biocide employée pour la préparation de la composition diluée selon l'invention comporte des composés provenant de ressources végétales, notamment agricoles, qui sont entièrement renouvelables. En conséquence, ladite formulation biocide est entièrement biodégradable.

Plus précisément, la composition est biodégradable à 100% selon les résultats des essais relatifs au protocole expérimental de l'OCDE.

En plus d'un impact significativement réduit sur l'environnement par rapport à l'état de l'art, les formulations biocides employées pour la préparation de la composition diluée selon l'invention présentent un effet innovant de mise à disposition biologique de l'actif biocide grâce à des structures tensioactives adaptées et sous l'effet de modifications physico-chimiques du mélange, ce qui se traduit par une combinaison recherchée entre les constituants et des performances permettant de substituer, à équi-concentration, les produits chimiques et dangereux du marché.

La présente formulation biocide présente une action efficace à l'encontre de certains organismes indésirables ou pathogènes, et un effet amélioré par rapport aux formulations biocides connues de l'art antérieur. L'action de la formulation est illustrée dans les exemples ci-dessous.

En particulier, cette formulation biocide permet de lutter contre des champignons appartenant à l'espèce *Aspergillus niger,* qui est susceptibles d'être toxique et pathogène, notamment en provoquant des mycoses pulmonaires chez l'homme.

La composition selon l'invention présente en outre une activité biocide, notamment virucide, à l'encontre de souches de virus appartenant à la famille des adénovirus, ceux-ci pouvant être responsables de maladies comme la pharyngite, les pneumonies, ou encore les conjonctivites. L'effet virucide a également été démontré à l'encontre de

rotavirus, responsables notamment de gastro-entérites.

En ce qui concerne les acides organiques utilisables dans la composition des formulations biocides selon la présente invention, on citera, notamment, l'acide citrique, l'acide lactique, l'acide succinique, l'acide pyruvique, l'acide glycolique.

Le choix de l'acide organique est fait en fonction du domaine d'application des formulations biocides et des organismes cibles à éliminer ou dont on souhaite ralentir le développement. Dans un mode de réalisation particulier, le ou les acides organiques sont l'acide lactique et l'acide citrique.

Dans un mode de réalisation encore plus préféré, seul l'acide lactique est utilisé.

En effet, l'acide lactique est l'un des éléments indispensables du métabolisme humain, mais également du métabolisme des animaux et des micro-organismes. L'acide lactique présente de façon intrinsèque des propriétés d'interférence sur les micro-organismes car il agit directement sur leur pH ainsi que sur leur cycle énergétique. Par ailleurs, il a également une action antimicrobienne en inhibant leur croissance (pour *Escherichia Coli* entre autres).

En plus de l'acide organique, la formulation biocide concentrée selon l'invention comporte également au moins un tensioactif anionique.

Celui-ci est avantageusement choisi parmi les carboxylates de sodium comprenant un nombre d'atomes de carbone compris entre 5 et 16.

Le tensioactif anionique peut également consister en un carboxylate de potassium comprenant un nombre d'atomes de carbone compris entre 5 et 16.

De préférence, la chaine carbonée des tensioactifs anioniques est issue d'alcool gras ou d'acide gras végétaux.

Ainsi, selon l'invention, le tensioactif anionique consiste en un alkyl carboxylate de type caprate de sodium ou caprylate de sodium.

Dans un exemple non couvert par les revendications, le tensioactif anionique peut être le laurate de sodium, C8-C16 alkyl sulfate de sodium, d'ammonium ou de potassium, un ester d'acide gras carboxylé.

Le tensioactif anionique peut également être constitué par les espèces acides issues de ces sels.

La formulation biocide concentrée employée pour la préparation de la composition diluée selon l'invention peut également comporter d'autres ingrédients.

Ainsi, ladite formulation peut comporter, en plus de l'acide organique et du tensioactif anionique, au moins un tensioactif non-ionique et/ou au moins un parfum naturel et/ou au moins un agent chélatant.

Les tensioactifs non-ioniques ou autres composants de type huiles essentielles ou chélatant pouvant entrer dans la composition du produit biocide selon l'invention sont sélectionnés en fonction du type de performance et d'application demandée : un judicieux mélange est nécessaire afin de ne pas perturber ou modifier les propriétés biocides recherchées initialement. Effectivement, par exemple, des propriétés désinfectantes performantes et/ou dégraissantes sont nécessaires dans l'univers de la restauration ou de l'agro-industrie avec des produits peu ou pas toxiques afin d'éviter toute contamination chimique préoccupante, notamment par la présence de résidus chimiques sur les surfaces après la procédure de désinfection.

Dans un mode de réalisation particulier, la formulation biocide employée pour la préparation de la composition diluée selon l'invention peut comporter au moins un tensioactif non-ionique, de préférence dans une proportion comprise entre 2 et 15% en masse par rapport à la masse totale de la formulation.

De manière avantageuse, ce tensioactif non-ionique est choisi parmi les glycosides et les alkyl polyglycosides dont la chaîne carbonée comporte entre 5 et 20 atomes de carbone, plus avantageusement encore entre 5 et 18 atomes de carbone et le groupement hydrophile est préférentiellement composé de résidus pentose ou hexose.

Ainsi, de préférence, la formulation employée pour la préparation de la composition diluée selon l'invention peut comporter un tensioactif non-ionique sélectionné parmi les octyl glucosides, les décyl glucosides, les lauryl glucosides, les coco glucosides, les D-glucopyranose, D-xylopyranose C10-C16 alkylpolyglycosides.

Les agents chélatants pouvant entrer dans la composition de la formulation biocide selon l'invention sont des agents conventionnels dans le domaine du nettoyage et de la désinfection des surfaces à usage domestique ou professionnel.

Ils sont sélectionnés parmi les chélatants couramment utilisés dans les produits biocides, tels que l'acide sorbique, issu du sorbier, ou l'acide oxalique, issu des racines ou des rhizomes de nombreuses plantes (oseille, betterave, etc.), les monomères ou polymères séquestrants comme les extraits de chicorée, ou des dérivés d'acides aminés.

En outre, l'agent chélatant peut également, dans un mode de réalisation non couvert par le texte des revendications, être l'acide citrique, issu du jus de citron ou de la fermentation de sucres végétaux simples ou complexes, notamment le glucose, le fructose, et le saccharose, ou issu de la fermentation de matériel agricole. Ce matériel agricole de base peut consister par exemple en des résidus de mélasse permettant d'obtenir des agents complexants de type gluconates.

Préférentiellement, le ou les agents chélatants sont présents à raison de 0,1% à 10% en masse par rapport à la masse totale de la formulation.

Les propriétés olfactives des produits biocides selon l'invention peuvent être conférées par la présence d'au

moins un parfum naturel issu d'une ressource naturelle renouvelable et biodégradable.

Ce parfum naturel peut être avantageusement choisi parmi les huiles essentielles, les essences végétales ou encore les extraits végétaux.

On citera notamment à titre d'exemple la menthe A, la menthe N ou l'eucalyptus G.

Préférentiellement, le ou les parfums naturels sont présents dans la formulation biocide concentrée à raison de 0,01% à 10% en masse par rapport à la masse totale de la formulation.

La présence du parfum apportera avantageusement une note olfactive, sans participer à l'activité biocide du produit. Ainsi, celui-ci pourra être retiré dans des conditions où des formulations sans parfum s'imposent, telles que dans l'industrie agroalimentaire.

Les formulations sont conformes à la réglementation européenne en termes d'efficacité. Elles répondent aux normes EN1040, EN1276, EN1656, EN 1275, EN 13697, EN1650 et EN14476+A1 en conditions de saleté ou de propreté.

En outre, ces formulations efficaces sont sans danger particulier pour l'Homme et sans préjudice pour l'Environnement.

Les formulations biocides employée pour la préparation de la composition diluée selon l'invention sont susceptibles d'être utilisées à des taux de dilution et dans des temps d'action comparables à ceux requis par le marché professionnel. En outre, elles sont conformes aux exigences d'efficacité biocide requises, sans danger particulier à l'application, biodégradables et sans écotoxicité après élimination.

Ainsi, une composition liquide aqueuse peut comprendre au moins la formulation biocide décrite plus haut et de l'eau, et toutes les dilutions dans l'eau de ladite composition jusqu'à une concentration minimale de 0,2% de ladite composition. Un tel exemple n'est pas couvert par le texte des revendications mais est considéré comme utile à la compréhension de l'invention.

Le restant de la composition comporte au moins de l'eau. Ainsi, dans un mode de réalisation particulier, la composition liquide aqueuse selon l'invention peut également comporter d'autres ingrédients de manière à améliorer les propriétés de nettoyage ou/et organoleptiques, sans toutefois modifier l'activité biocide de ladite formulation.

Dans un exemple de réalisation non couvert par le texte des revendications, la formulation biocide selon l'invention est présente, dans ladite composition liquide aqueuse, dans une proportion comprise entre 0,2 et 2% en masse, par rapport à la masse totale de ladite composition.

En d'autres termes, ladite formulation peut être diluée avantageusement entre 50 et 500 fois et être toujours active sur les organismes indésirables que l'on souhaite éliminer.

La formulation biocide selon l'invention est présente, dans ladite composition liquide aqueuse, dans une proportion comprise entre 1 et 2% en masse, plus avantageusement entre 1,5 et 2% en masse, par rapport à la masse totale de ladite composition.

Les exemples ci-après permettent d'illustrer cette efficacité, notamment à l'encontre de certaines souches pathogènes ou indésirables de levures, de champignons et de bactéries, mais également à l'encontre de certains virus pathogènes.

En particulier, les formulations biocides sont particulièrement intéressantes car elles restent toujours efficaces même à des taux de dilution élevés, et présentent une efficacité comparable à des formulations plus concentrées mais qui sont moins intéressantes d'un point de vue économique.

Les applications envisagées pour les formulations biocides selon l'invention sont basées sur une action désinfectante mais aussi détergente, dégraissante, émolliente, olfactive, etc...

On citera à titre d'exemples, mais sans que cette liste ne soit exhaustive, une application des formulations selon l'invention à des produits anti-mousse pour nettoyer les ouvrages de maçonnerie ; des produits pour nettoyer les pontons et les coques de bateaux des mousses et algues qui les colonisent ; des solutions détergentes désinfectantes pour tous types de surface ; des solutions pour l'hygiène des mains par lavage ou par friction ; des solutions de désinfection des denrées alimentaires ; des solutions pour la désinfection de locaux, bâtiments et matériels de transport et d'élevage ; des solutions de désinfection des surfaces pour l'industrie agroalimentaire, cosmétique ou pharmaceutique ; des solutions de désinfection pour locaux et/ou transport des ordures ménagères, des solutions de désinfection du matériel de traite, des trayons, des litières d'animaux.

Dans un mode de réalisation particulier, les formulations biocides employées pour la préparation de la composition diluée selon l'invention sont utilisées pour l'hygiène des mains, par lavage ou par friction, sous forme liquide ou semi-liquide, avec ou sans rinçage à l'eau.

La présente description décrit également sur un substrat imprégné avec une formulation biocide concentrée employée pour la préparation de la composition diluée selon l'invention, permettant l'application de ladite formulation sur une surface à nettoyer et à désinfecter.

Ce substrat peut consister en un tissu, une lingette ou une serviette.

Sur son substrat, la formulation peut être pure et déshydratée et n'être activée qu'une fois en présence d'eau ou encore être déjà diluée et prête à l'emploi.

En d'autres termes, le substrat peut également être imprégné de la composition liquide aqueuse telle que décrite

ci-dessus, ladite composition comportant au moins la formulation biocide et de l'eau, ladite formulation étant présente dans une proportion comprise entre 0,2% et 2% en masse par rapport à la masse totale de ladite composition.

Les modes de réalisation cités ci-dessus et concernant un substrat imprégné d'une formulation pure ou d'une composition liquide aqueuse avec la formulation présente dans une proportion comprise entre 0,2 et 2 % ne sont pas couverts par le texte des revendications, mais sont considérés comme utiles à la compréhension de l'invention.

Exemple de formulation biocide:

Dans un exemple de réalisation, la formulation biocide employée pour la préparation de la composition diluée selon l'invention présente les composés suivants, dans les proportions suivantes :

- 2,4% de tensioactifs non-ioniques de type glycosides ;
- 5% de tensioactifs anioniques de type sels d'acides gras avec une chaîne contenant 8 à 14 atomes de carbone de type cocoate de sodium;
- 91% d'acide lactique
- 1,6% d'eau.

Cette formule est uniquement classée irritante selon la réglementation en vigueur, au même titre que l'acide lactique utilisé dans ladite formulation. Un tel classement lui permet de passer au travers de dossiers d'instruction lourds et couteux pour une mise sur le marché. En outre, cette formulation ne présente aucun danger pour l'homme ou pour l'environnement comparativement aux substances d'origines chimiques qui sont commercialisées sur le marché.

Cette formulation est diluée en fonction des surfaces à nettoyer, à assainir et de l'efficacité désinfectante recherchée. La formule diluée active permettant une désinfection de surface ne présente aucun danger connu et/ou règlementé.

Dans un deuxième exemple de réalisation, la formulation biocide employée pour la préparation de la composition diluée selon l'invention est composée comme suit :

- 76% d'acide lactique ;
- 24% de tensioactifs anioniques de type sels d'acides gras avec une chaîne contenant 8 à 10 atomes de carbone de type alkyl carboxylate de sodium.

Dans un troisième exemple de réalisation, la formulation biocide employée pour la préparation de la composition diluée selon l'invention est composée comme suit :

- 80% d'acide lactique ;
- 5% de tensioactifs anioniques de type sels d'acides gras de type cocoate de sodium;
- 1% de composition parfumante à base d'extraits végétaux ;
- 14% eau.

Dans un quatrième exemple de réalisation, la formulation biocide employée pour la préparation de la composition diluée selon l'invention est composée comme suit :

- 76% d'acide lactique ;
- 10% de tensioactif non-ioniques de type alkyl polyglycosides ;
- 10% de tensioactif anioniques de type C10-C12 carboxylate de sodium ;
- 4% eau.

Tests d'efficacité de la formulation biocide

La formulation biocide qui a été testée est celle qui est précisée ci-dessus et qui comporte :

- 2,4% de tensioactifs non-ioniques de type C10-C16 alkyl polyglycosides ;
- 5% de tensioactifs anioniques de type sels d'acides gras type caprylate de sodium;
- 91% d'acide lactique ;
- 1,6% eau.

1. Levures - *Candida albicans*

Cet essai quantitatif de suspension pour l'évaluation de l'activité levuricide de base de la formulation selon l'invention est réalisé par un Laboratoire de Microbiologie. Cet essai est réalisé en conditions de saleté et en conditions additionnelles.

Méthode : Une méthode de dilution/neutralisation est appliquée à une formulation selon l'invention. Le neutralisant est constitué de chlorure de sodium (0,85% P/V), de $Na_2CO_3$ (0,1%), complété par de l'eau distillée.

La formulation employée pour la préparation de la composition diluée selon l'invention est diluée dans de l'eau physiologique aux concentrations suivantes : 1%, 1,2%, 1,5% et 2%.

Les tests sont réalisés sur une souche de *Candida albicans* (ATCC 90028) à une température de 20° C (± 1°C). Le temps de contact est limité à 5 minutes (± 10 secondes).

La substance interférente est de l'albumine bovine (fraction V) à raison de 3,00 g/l. Ceci constitue la condition de saleté.

Les tests sont incubés à une température de 25,0°C (± 0,2°C) pendant 48h.

Résultats : conformément aux dispositions de la norme NF EN 1275 (Avril 2006) la formule selon l'invention présente une activité levuricide en conditions de saleté (3,00 g/l d'albumine bovine) après 5 minutes de contact à 20°C vis-à-vis d'une souche de référence de *Candida albicans* pour la concentration de 1,2%.

2. Champignons - *Aspergillus niger*

Il a également été testé l'activité fongicide de la formulation biocide employée pour la préparation de la composition diluée selon l'invention, plus particulièrement sur un champignon appartenant à l'espèce *Aspergillus niger.*

La formulation biocide est diluée à une concentration de 2% dans de l'eau.

Les essais ont été conduits à une température de 20°C (± 1°C). Deux temps de contact ont été testés : 15 min et 30 min.

La substance interférente est de l'albumine bovine (fraction V) à raison de 0,30 g/l. Ceci constitue la condition de propreté.

Les tests sont incubés à une température de 30°C pendant une durée de 2 jours.

Résultats : La formulation diluée à 2% présente une activité fongicide à l'encontre de la souche *Aspergillus niger* après un temps de contact de 30 min à une température de 20°C et en conditions de propreté (0,30g/l d'albumine bovine).

Les résultats obtenus pour *C. albicans* et *A. niger* sont repris dans le tableau ci-dessous.

Tableau 1. Essais d'activité fongicide selon EN1650, conditions de saleté (3 g/L de BSA) et de propreté (0,3g/L de BSA), 20°C

| Souche | Lot testé | Concentration d'essai (w/w) | Temps de contact | Réduction log | Activité fongicid e | BSA (g/L) |
|---|---|---|---|---|---|---|
| ***Candida albicans*** | 0182/2013 | 2% | 5 min | R>4, 22 | OUI | 3 |
| | | 1,5% | 5 min | R>4, 22 | OUI | 3 |
| | | 1,2% | 5 min | R > 4,07 | OUI | 3 |
| | | 1% | 5 min | R<2, 70 | NON | 3 |
| ***Aspergillus niger*** | 0346A/2013 | 2% | 30 min | R = 4,125 | OUI | 0,3 |
| | 0395A/2013 | 2% | 15 min | R = 4,25 | NON | 0,3 |

3. Virus - Rotavirus et Adénovirus

Ce test d'efficacité virucide est réalisé selon la méthodologie de la norme NF EN 14476+A1 (Janvier 2007) sur deux virus.

a) Rotavirus

Le premier virus testé est un Rotavirus (souche ATCC VR-1290) et les essais ont été conduits sur 3 échantillons de produit désinfectant prêts à l'emploi, selon la formulation diluée à 2% dans de l'eau.

Méthode : Le test est réalisé à 20°C (± 1°C). Le temps de contact est de 15 minutes. Les échantillons sont dilués dans de l'eau distillée stérile. La substance interférente est de l'albumine bovine (BSA 3,00 g/l).

La souche virale a été testée sur des cellules MA-104 à 36,5°C sous 5% de $CO_2$.

La technique d'arrêt de l'action virucide est l'adjonction d'une solution contenant $Na_2CO_3$, à raison de 70 g/l, pH 10,85 et 10ml de solution d'arrêt pour 10 ml de solution d'essai. Une telle solution permet de stopper l'action du désinfectant sur le virus et de mesurer l'efficacité de la formulation biocide testée.

Le virus est titré en UFP/ml (unités formant plage) soit en nombre de particules virales infectieuses par ml et plus communément en logarithme du titre viral.

Résultats Les trois échantillons de formulation biocide diluées ont une activité virucide sur la souche de Rotavirus à une concentration de 2% pour 15 min de contact.

b) Adénovirus

Le second virus testé est un Adénovirus (souche adénoïd 75 ATCC VR-5).

La formulation biocide employée pour la préparation de la composition diluée selon l'invention est diluée à 2% dans de l'eau et la méthode d'essai est la même que celle utilisée sur le Rotavirus et décrite ci-dessus.

Toutefois, le temps de contact est de 60 min.

La souche virale a été testée sur des cellules HEp-2 à 36,5°C sous 5% de $CO_2$.

Résultats Les trois échantillons de formulation biocide diluées selon l'invention ont une activité virucide sur la souche d'Adénovirus à une concentration de 2% pour 60 min de contact.

Les résultats obtenus pour les tests d'activité virucide sont repris dans le tableau ci-dessous :

Tableau 2. Essais d'activité virucide selon EN 14476+A1, conditions de propreté (0,3 g/L de BSA), 20°C

| Souche virale | Lot testé | Concentration d'essai (w/w) | Temps de contact | Réduction log | Activité virucide | BSA (g/L) |
|---|---|---|---|---|---|---|
| Rotavirus souche ATCC TVR-1290 | 0212/2013 | 2% | 15 min | R = 4,25 | OUI | 0,3 |
| | 0226/2013 | 2% | 15 min | R = 4,375 | OUI | 0,3 |
| | 0227/2013 | 2% | 15 min | R = 4, 125 | OUI | 0,3 |
| | | | | | | |
| Adénovirus souche adénoïd 75 ATCC VR-5 | 0395A/2013 | 2% | 60 min | R = 4,125 | OUI | 0,3 |
| | 0406A/2013 | 2% | 60 min | R = 4,25 | OUI | 0,3 |
| | 0406B/2013 | 2% | 60 min | R = 4,125 | OUI | 0,3 |

Il a également été démontré que la présente formulation biocide est efficace à une concentration de 1% sur les virus enveloppés à génome ADN, tels que ceux appartenant à la famille des *Herpesviridae.* En outre, la formulation biocide est également efficace à 1% sur des virus influenza.

4. Bactéries

Cet essai quantitatif de suspension pour l'évaluation de l'activité bactéricide des formulations employée pour la préparation de la composition diluée selon la présente invention est réalisé par un Laboratoire de Microbiologie.

Méthode : Une méthode de dilution/neutralisation est appliquée à une formulation employée pour la préparation de la composition diluée selon l'invention. Le neutralisant est constitué de chlorure de sodium (0,85% P/V), de $Na_2CO_3$ (0,1%), complété par de l'eau distillée. Cette méthode de dilution/neutralisation permet de stopper l'action biocide du produit à la fin du temps de contact testé.

**La** formulation employée pour la préparation de la composition diluée selon l'invention est diluée dans de l'eau physiologique aux concentrations suivantes : 0,5%, 1,2% et 1,5%. La dilution à 0,5% n'illustre pas une composition selon l'invention.

Les tests sont réalisés sur des souches de *Staphylococcus aureus* (ATCC 6538), *Escherichia coli* (ATCC 10536), *Enterococcus hirae* (ATCC 10541) et *Pseudomonas aeruginosa* (ATCC 15442) à une température de 20° C (± 1°C). Le temps de contact est limité à 5 minutes (± 10 secondes).

La substance interférente est de l'albumine bovine (fraction V) à raison de 3,00 g/l. Ceci constitue la condition de saleté.

L'incubation est effectuée à une température de 37,0°C (± 0,2°C) pendant 24h.

Résultats : Selon les indications de la norme NF EN 1276 (octobre 1997), une formule concentrée de désinfection selon la formulation de la présente invention, présente une activité bactéricide en conditions de saleté (3,00 g/l d'albumine

bovine) après 5 minutes de contact à 20°C vis-à-vis des 4 souches de référence testées pour la concentration test de 1%. Le microorganisme limitant est *Staphylococcus aureus* (ATCC 6538).

Les résultats des tests bactéricides sont représentés dans le tableau 3 ci-dessous.

| | test suspension | | test procedure at concentration of % (v/v) | | | |
|---|---|---|---|---|---|---|
| **Test organisms** | (N and $N_0$) | | | **0,5%** | **1,2%** | **1,5%** |
| *Pseudomonas aeruginosa* ATCC15442 | $10^{-6}$ | 291 | Vc | > 300 | 26 | <10 |
| | $10^{-7}$ | 22 | Na | > 3000 | 260 | <100 |
| | N | 2,85E+08 | Ig Na | 3,47 | 2,41 | 2,00 |
| | Ig N | 8,45 | R | <3,98 | 5,04 | >5,45 |
| | Ig $N_0$ | 7,45 | Active | NO | YES | YES |
| *Enterococcus hirae* ATCC 10541 | $10^{-6}$ | 122 | Vc | > 300 | <10 | <10 |
| | $10^{-7}$ | 12,0 | Na | > 3000 | <100 | <100 |
| | N | 1,20E+08 | Ig Na | 3,47 | <100 | <100 |
| | Ig N | 8,08 | R | <3,61 | >5,08 | >5,08 |
| | Ig $N_0$ | 7,08 | Active | NO | YES | YES |
| *Staphylococcus aureus* ATCC 6538 | $10^{-6}$ | 144 | Vc | > 300 | <10 | <10 |
| | $10^{-7}$ | 7,1 | Na | > 3000 | <100 | <100 |
| | N | 1,44E+08 | Ig Na | 3,47 | 2,00 | 2,00 |
| | Ig N | 8,16 | R | <3,69 | >5,16 | >5,16 |
| | Ig $N_0$ | 7,16 | Active | NO | YES | YES |
| *Escherichia coli* ATCC 10536 | $10^{-6}$ | 160 | Vc | > 300 | <10 | <10 |
| | $10^{-7}$ | 18 | Na | > 3000 | <100 | <100 |
| | N | 1,62E+08 | Ig Na | 3,47 | 2,00 | 2,00 |
| | Ig N | 8,21 | R | <3,74 | >5,21 | >5,21 |
| | Ig $N_0$ | 7,21 | Active | NO | YES | YES |

Tableau 3. Essais d'activité selon EN 1276, conditions de saleté (3 g/L de BSA), temps de contact 5min, 20°C

Des tests sont également réalisés selon une méthode normalisée (EN13697) de manière à démontrer l'effet bactéricide et/ou levuricide par application sur des surfaces.

Les surfaces témoins utilisées sont des disques en acier inoxydables sur lesquels est déposée une solution de bactéries et/ou de levures. Il s'agit plus particulièrement des souches microbiennes exposées ci-dessus. Les disques sont ensuite immergés dans la solution biocide à 20°C (+/- 0,2°C) pendant un temps de contact de 5 minutes. La concentration microbienne résiduelle est déterminée après incubation à 37°C (+/-2°C) pour les bactéries ou à 25°C (+/-0,2°C) pour les levures.

Résultats : Selon les indications de la norme EN 13697 (novembre 2001), une formule présente une activité bactéricide et levuricide en conditions de saleté (3,00 g/l d'albumine bovine) après 5 minutes de contact à 20°C vis-à-vis des 4 souches bactériennes de référence testées et de la souche levurienne pour la concentration test de 1%.

5. Biodégradabilité

Cet essai est une évaluation en milieu aqueux de la biodégradabilité aérobie ultime d'un échantillon de la formulation concentrée.

Cet essai est réalisé selon les dispositions de la méthode OCDE 301 A utilisant un échantillon contenant une teneur en carbone organique dissous de 10 mg/l (COD pour carbone organique dissous). L'évolution de la disparition du carbone organique dissous est suivi pendant une période de 28 jours dans l'essai « Désinfection concentré » à 10 mg/l de COD ». L'analyse physico-chimique est réalisée suivant la norme ASTM D5291.

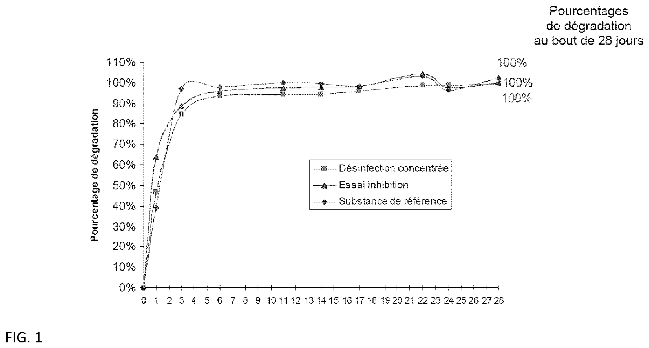

La figure 1 ci jointe représente les courbes de résultats de dégradation du carbone organique dissous au bout de 28 jours. La substance de référence correspond à de l'acétate de sodium. L'échantillon référencé « désinfection concentrée » correspond à la fomulation biocide testé décrite ci-dessus et l'échantillon « essai inhibition » correspond à un mélange entre la formulation et l'acétate de sodium.

Résultats :

L'essai est considéré comme valide car :

- Le pourcentage de dégradation de la substance de référence (acétate de sodium) est supérieur à 70 % (99 %) au quatorzième jour ;

- La différence entre les valeurs de mesures de COD du produit étudié est inférieure à 20% à chaque prélèvement ;
- Le degré d'élimination du COD dans l'essai inhibiteur, contenant le composé d'essai et la substance de référence, étant supérieur à 35% au bout de 14 jours (98%), l'échantillon n'est pas considéré comme toxique vis-à-vis de l'ensemencement.

Conclusion :

L'évaluation en milieux aqueux de la biodégradabilité "ultime" de l'échantillon testé suivant la ligne directrice OCDE 301 A donne les résultats suivant :

- Le degré de biodégradation maximal de l'échantillon à 10 mg/l de COD est égal à 100% après 28 jours d'incubation ;
- Le temps de biodégradation (correspondant à 90 % du taux de biodégradation maximal) est d'environ 25 jours ;
- D'après la ligne directrice OCDE 301 A, l'échantillon testé « Désinfection concentrée » est considéré comme facilement biodégradable (le niveau de seuil de diminution du COD de 70% étant acquis dans un intervalle de 10 jours après que 10% de biodégradabilité soit atteint (94% à la fin de l'intervalle de 10 jours au 11$^{ème}$ jour de test).

Le degré de biodégradabilité mesuré prend en compte la totalité de la formulation : effectivement il n'y a pas de sous-produit de dégradation car la formulation est exclusivement composée d'atomes de carbone, d'oxygène et d'hydrogène provenant d'extraits végétaux. C'est une mesure vraie de biodégradabilité ultime.

## Revendications

**1.** Composition liquide aqueuse à effet virucide à l'encontre de souches de virus appartenant à la famille des adénovirus ou à effet virucide à l'encontre de rotavirus ou à effet fongicide à l'encontre de champignons appartenant à l'espèce *Aspergillus niger,* ladite composition comprenant une formulation biocide concentrée biodégradable, comportant des composés provenant de ressources végétales renouvelables, ladite formulation biocide concentrée étant **caractérisée en ce qu'**elle comporte au moins les composés suivants :

- au moins de l'acide lactique dans une proportion comprise entre 76 et 98 % en masse, par rapport à la masse totale de la formulation ;
- au moins un tensioactif anionique consistant en un alkyl carboxylate sélectionné parmi le caprate de sodium et le caprylate de sodium, dans une proportion comprise entre 2 et 24 % en masse, par rapport à la masse totale de la formulation ;

ladite formulation biocide étant présente, dans ladite composition liquide aqueuse, dans une proportion comprise entre 1 et 2% en masse, par rapport à la masse totale de ladite composition, le restant de ladite composition étant de l'eau.

**2.** Composition liquide aqueuse selon la revendication précédente dans laquelle ladite formulation biocide est présente dans une proportion comprise entre 1,5 et 2% par rapport à la masse totale de ladite composition liquide aqueuse.

**3.** Composition liquide aqueuse selon l'une quelconque des revendications précédentes dans laquelle ladite formulation biocide est présente dans une proportion égale à 2% par rapport à la masse totale de ladite composition liquide aqueuse.

**4.** Composition liquide aqueuse selon l'une quelconque des revendications précédentes dans laquelle ladite formulation biocide concentrée comporte entre 76 et 92% en masse d'acide lactique et entre 3 et 15% en masse dudit tensioactif anionique consistant en un alkyl carboxylate sélectionné parmi le caprate de sodium et le caprylate de sodium.

**5.** Composition liquide aqueuse selon l'une quelconque des revendications précédentes dans laquelle ladite formulation biocide comporte entre 5 et 10% en masse de tensioactif anionique consistant en un alkyl carboxylate sélectionné parmi le caprate de sodium et le caprylate de sodium.

**6.** Composition liquide aqueuse selon l'une quelconque des revendications précédentes dans laquelle ladite formulation biocide concentrée comprend également un tensioactif non-ionique dans une proportion comprise entre 2 et 15% en masse, par rapport à la masse totale de la formulation, ledit tensioactif non-ionique étant choisi parmi les

glycosides et les alkyl polyglycosides comprenant un nombre d'atomes de carbone compris entre 5 et 20, le groupement hydrophile étant composé de pentose ou d'hexose.

**7.** Composition liquide aqueuse selon la revendication précédente dans laquelle ladite formulation biocide concentrée comprend un tensioactif non-ionique avec entre 5 et 18 atomes de carbone.

**8.** Composition liquide aqueuse selon l'une quelconque des revendications 6 ou 7 dans laquelle ladite formulation biocide concentrée comprend un tensioactif non-ionique sélectionné parmi les octyl glucosides, les décyl glucosides, les lauryl glucosides, les coco glucosides, le D-glucopyranose, le D-xylopyranose ou les C10-C16 alkylpolyglycosides.

**9.** Composition liquide aqueuse selon l'une quelconque des revendications précédentes dans laquelle ladite formulation biocide concentrée comprend également un parfum naturel dans une proportion comprise entre 0,01 et 10% en masse, par rapport à la masse totale de la formulation, ledit parfum étant choisi parmi les huiles essentielles, les essences végétales et les extraits végétaux.

**10.** Composition liquide aqueuse selon l'une quelconque des revendications précédentes dans laquelle ladite formulation biocide comprend également un agent chélatant dans une proportion comprise entre 0,1 et 10% en masse, par rapport à la masse totale de la composition, ledit agent chélatant étant choisi parmi l'acide sorbique issu du sorbier, l'acide oxalique issu des racines ou des rhizomes de plantes, les monomères ou polymères séquestrant, et les acides aminés.

**11.** Composition liquide aqueuse selon l'une quelconque des revendications 1, 2 et 4 à 10 dans laquelle le tensioactif anionique de ladite formulation biocide concentrée consiste en du caprylate de sodium.

**12.** Composition liquide aqueuse selon l'une quelconque des revendications précédentes dans laquelle ladite formulation biocide concentrée comporte :

- 2,4% de tensioactifs non-ioniques de type C10-C16 alkyl polyglycosides ;
- 5% de tensioactifs anioniques de type sels d'acides gras de type caprylate de sodium ;
- 91% d'acide lactique ;
- 1,6% d'eau,

ladite formulation biocide étant présente, dans ladite composition liquide aqueuse, dans une proportion égale à 2% en masse, par rapport à la masse totale de ladite composition, le restant de ladite composition étant de l'eau.

**13.** Procédé non-thérapeutique d'inactivation de rotavirus **caractérisé en ce qu'**on applique la composition liquide aqueuse selon la revendication précédente pendant un temps de contact de 15 min, à une température de 20°C (+/- 1°C).

**14.** Procédé non-thérapeutique d'inactivation de souches de virus appartenant à la famille des adénovirus **caractérisé en ce qu'**on applique la composition liquide aqueuse selon la revendication 12 pendant un temps de contact de 60 min, à une température de 20°C (+/-1°C) .

**15.** Procédé non-thérapeutique d'inactivation de souches d'un champignon appartenant à la souche *Aspergillus niger* **caractérisé en ce qu'**on applique la composition liquide aqueuse selon la revendication 12 pendant un temps de contact de 30 min, à une température de 20°C (+/- 1°C).

## Patentansprüche

**1.** Wässrige flüssige Zusammensetzung mit viruzider Wirkung gegen Virusstämme der Familie der Adenoviren oder mit viruzider Wirkung gegen Rotaviren oder mit fungizider Wirkung gegen Pilze der Art Aspergillus niger, wobei die Zusammensetzung eine biologisch abbaubare, konzentrierte Biozidformulierung umfasst, die Verbindungen aus nachwachsenden pflanzlichen Rohstoffen enthält, wobei die konzentrierte Biozidformulierung **dadurch gekennzeichnet ist, dass** sie wenigstens die folgenden Verbindungen umfasst:

- wenigstens Milchsäure in einem Anteil zwischen 76 und 98 Massenprozent, bezogen auf die Gesamtmasse der

Formulierung;
- wenigstens ein anionisches Tensid, bestehend aus einem Alkylcarboxylat, gewählt aus Natriumcaprat und Natriumcaprylat, in einem Anteil zwischen 2 und 24 Massenprozent, bezogen auf die Gesamtmasse der Formulierung;

wobei die biozide Formulierung in der wässrigen flüssigen Zusammensetzung in einem Anteil zwischen 1 und 2 Massenprozent, bezogen auf die Gesamtmasse der Zusammensetzung, vorliegt, wobei der Rest der Zusammensetzung Wasser ist.

**2.** Wässrige flüssige Zusammensetzung nach dem vorhergehenden Anspruch, wobei die Biozidformulierung in einem Anteil zwischen 1,5 und 2 % bezogen auf die Gesamtmasse der wässrigen flüssigen Zusammensetzung vorliegt.

**3.** Wässrige flüssige Zusammensetzung nach einem der vorhergehenden Ansprüche, wobei die Biozidformulierung in einem Anteil von 2 % bezogen auf die Gesamtmasse der wässrigen flüssigen Zusammensetzung vorliegt.

**4.** Wässrige flüssige Zusammensetzung nach einem der vorhergehenden Ansprüche, wobei die konzentrierte biozide Formulierung zwischen 76 und 92 Massenprozent Milchsäure und zwischen 3 und 15 Massenprozent des anionischen Tensids umfasst, das aus einem Alkylcarboxylat besteht, das aus Natriumcaprat und Natriumcaprylat gewählt ist.

**5.** Wässrige flüssige Zusammensetzung nach einem der vorhergehenden Ansprüche, wobei die Biozidformulierung zwischen 5 und 10 Massenprozent eines anionischen Tensids umfasst, das aus einem Alkylcarboxylat besteht, das aus Natriumcaprat und Natriumcaprylat gewählt ist.

**6.** Wässrige flüssige Zusammensetzung nach einem der vorhergehenden Ansprüche, wobei die konzentrierte biozide Formulierung ferner ein nichtionisches Tensid in einem Anteil zwischen 2 und 15 Massenprozent, bezogen auf die Gesamtmasse der Formulierung, umfasst, wobei das nichtionische Tensid gewählt ist aus Glykosiden und Alkylpolyglykosiden, die eine Kohlenstoffatomzahl zwischen 5 und 20 aufweisen, wobei die hydrophile Gruppe aus einer Pentose oder einer Hexose besteht.

**7.** Wässrige flüssige Zusammensetzung nach dem vorhergehenden Anspruch, wobei die konzentrierte biozide Formulierung ein nichtionisches Tensid mit 5 bis 18 Kohlenstoffatomen enthält.

**8.** Wässrige flüssige Zusammensetzung nach einem der Ansprüche 6 oder 7, wobei die konzentrierte biozide Formulierung ein nichtionisches Tensid umfasst, das gewählt ist aus Octylglucosiden, Decylglucosiden, Laurylglucosiden, Kokosglucosiden, D-Glucopyranose, D-Xylopyranose oder C10-C16-Alkylpolyglykosiden.

**9.** Wässrige flüssige Zusammensetzung nach einem der vorhergehenden Ansprüche, wobei die konzentrierte biozide Formulierung ferner einen natürlichen Duftstoff in einem Anteil zwischen 0,01 und 10 Massenprozent, bezogen auf die Gesamtmasse der Formulierung, umfasst, wobei der Duftstoff aus ätherischen Ölen, Pflanzenessenzen und Pflanzenextrakten gewählt ist.

**10.** Wässrige flüssige Zusammensetzung nach einem der vorhergehenden Ansprüche, wobei die Biozidformulierung ferner einen Chelatbildner in einem Anteil zwischen 0,1 und 10 Massenprozent, bezogen auf die Gesamtmasse der Zusammensetzung, umfasst, wobei der Chelatbildner gewählt ist aus Sorbinsäure aus der Eberesche, Oxalsäure aus den Wurzeln oder Rhizomen von Pflanzen, sequestrierende Monomere oder Polymere sowie Aminosäuren gewählt ist.

**11.** Wässrige flüssige Zusammensetzung nach einem der Ansprüche 1, 2 und 4 bis 10, wobei das anionische Tensid der konzentrierten bioziden Formulierung aus Natriumcaprylat besteht.

**12.** Wässrige flüssige Zusammensetzung nach einem der vorhergehenden Ansprüche, wobei die konzentrierte biozide Formulierung umfasst:

- 2,4 % nichtionische Tenside vom Typ C10-C16-Alkylpolyglykoside;
- 5 % anionische Tenside vom Typ Fettsäuresalze vom Typ Natriumcaprylat;
- 91 % Milchsäure;
- 1,6 % Wasser,

wobei die biozide Formulierung in der wässrigen flüssigen Zusammensetzung in einem Anteil von 2 Massenprozent, bezogen auf die Gesamtmasse der Zusammensetzung, vorliegt und der Rest der Zusammensetzung Wasser ist.

**13.** Nichttherapeutisches Verfahren zur Inaktivierung von Rotaviren, **dadurch gekennzeichnet, dass** die wässrige flüssige Zusammensetzung nach dem vorhergehenden Anspruch während einer Einwirkzeit von 15 min bei einer Temperatur von 20 °C (+/-1 °C) angewendet wird.

**14.** Nichttherapeutisches Verfahren zur Inaktivierung von Virusstämmen, die zur Familie der Adenoviren gehören, **dadurch gekennzeichnet, dass** die wässrige flüssige Zusammensetzung nach Anspruch 12 während einer Einwirkzeit von 60 Minuten bei einer Temperatur von 20 °C (+/- 1 °C) aufgebracht wird.

**15.** Nichttherapeutisches Verfahren zur Inaktivierung von Pilzstämmen, die zum Stamm Aspergillus niger gehören, **dadurch gekennzeichnet, dass** die wässrige flüssige Zusammensetzung nach Anspruch 12 während einer Einwirkzeit von 30 Minuten bei einer Temperatur von 20 °C (+/- 1 °C) angewendet wird.

## Claims

**1.** Aqueous liquid composition with virucidal effect against virus strains belonging to the adenovirus family or with virucidal effect against rotavirus or with fungicidal effect against fungi belonging to the species *Aspergillus niger,* said composition comprising a biodegradable concentrated biocidal formulation, comprising compounds obtained from renewable plant resources, said concentrated biocidal formulation being **characterised in that** it comprises at least the following compounds:

- at least lactic acid in a proportion of between 76 and 98% by mass relative to the total mass of the formulation;
- at least one anionic surfactant consisting of an alkyl carboxylate selected from sodium caprate and sodium caprylate in a proportion of between 2 and 24% by mass relative to the total mass of the formulation;

said biocidal formulation being present, in said aqueous liquid composition, in a proportion of between 1 and 2% by mass relative to the total mass of said composition, the remainder of said composition being water.

**2.** Aqueous liquid composition according to the preceding claim, wherein said biocidal formulation is present in a proportion of between 1.5 and 2% relative to the total mass of said aqueous liquid composition.

**3.** Aqueous liquid composition according to any one of the preceding claims, wherein said biocidal formulation is present in a proportion equal to 2% relative to the total mass of said aqueous liquid composition.

**4.** Aqueous liquid composition according to any one of the preceding claims, wherein said concentrated biocidal formulation comprises between 76 and 92% by mass of lactic acid and between 3 and 15% by mass of said anionic surfactant consisting of an alkyl carboxylate selected from sodium caprate and sodium caprylate.

**5.** Aqueous liquid composition according to any one of the preceding claims, wherein said biocidal formulation comprises between 5 and 10% by mass of anionic surfactant consisting of an alkyl carboxylate selected from sodium caprate and sodium caprylate.

**6.** Aqueous liquid composition according to any one of the preceding claims, wherein said concentrated biocidal formulation also comprises a nonionic surfactant in a proportion of between 2 and 15% by mass relative to the total mass of the formulation, said nonionic surfactant being chosen from glycosides and alkyl polyglycosides comprising a number of carbon atoms between 5 and 20, the hydrophilic group being composed of pentose or hexose.

**7.** Aqueous liquid composition according to the preceding claim, wherein said concentrated biocidal formulation comprises a nonionic surfactant with between 5 and 18 carbon atoms.

**8.** Aqueous liquid composition according to any one of claims 6 or 7, wherein said concentrated biocidal formulation comprises a nonionic surfactant selected from octyl glucosides, decyl glucosides, lauryl glucosides, coco glucosides, D-glucopyranose, D-xylopyranose or C10-C16 alkyl polyglycosides.

**9.** Aqueous liquid composition according to any one of the preceding claims, wherein said concentrated biocidal

formulation also comprises a natural fragrance in a proportion of between 0.01 and 10% by mass relative to the total mass of the formulation, said fragrance being chosen from essential oils, plant essences and plant extracts.

**10.** Aqueous liquid composition according to any one of the preceding claims, wherein said biocidal formulation also comprises a chelating agent in a proportion of between 0.1 and 10% by mass relative to the total mass of the composition, said chelating agent being chosen from sorbic acid derived from rowan, oxalic acid derived from plant roots or rhizomes, sequestering monomers or polymers, and amino acids.

**11.** Aqueous liquid composition according to any one of claims 1, 2 and 4 to 10, wherein the anionic surfactant of said concentrated biocidal formulation consists of sodium caprylate.

**12.** Aqueous liquid composition according to any one of the preceding claims, wherein said concentrated biocidal formulation comprises:

- 2.4% C10-C16 alkyl polyglycoside type nonionic surfactants;
- 5% fatty acid salt type anionic surfactants of the sodium caprylate type;
- 91% lactic acid;
- 1.6% water,

said biocidal formulation being present, in said aqueous liquid composition, in a proportion equal to 2% by mass relative to the total mass of said composition, the remainder of said composition being water.

**13.** Non-therapeutic method for inactivating rotavirus, **characterised in that** the aqueous liquid composition according to the preceding claim is applied for a contact time of 15 min at a temperature of 20°C (+/- 1°C).

**14.** Non-therapeutic method for inactivating virus strains belonging to the adenovirus family, **characterised in that** the aqueous liquid composition according to claim 12 is applied for a contact time of 60 min at a temperature of 20°C (+/- 1°C).

**15.** Non-therapeutic method for inactivating strains of a fungus belonging to the *Aspergillus niger* strain, **characterised in that** the aqueous liquid composition according to claim 12 is applied for a contact time of 30 min at a temperature of 20°C (+/-1°C).

Pourcentages
de dégradation
au bout de 28 jours
100%
100%
100%
Pourcentage de dégradation
110%
100%
90%
80%
70%
60%
50%
40%
30%
20%
10%
0%
Désinfection concentrée
Essai inhibition
Substance de référence
0 1 2 3 4 5 6 7 8 9 10 11 12 13 14 15 16 17 18 19 20 21 22 23 24 25 26 27 28

FIG. 1

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 2012114039 A